# EUROPEAN PATENT APPLICATION

(11) **EP 4 649 908 A1**
(43) Date of publication of application: **19.11.2025**
(21) Application number: 25175896.7
(22) Date of filing: 13.05.2025
(51) Int. Cl.: A61B 34/00, A61B 34/10, A61B 34/20, A61B 18/14

(54) **ABLATION LOCATION PREDICTION OVER ANATOMICAL MAPS**

(30) Priority: 14.05.2024 US 202418663246
(71) Applicant: Biosense Webster (Israel) Ltd., 2066717 Yokneam (IL)
(72) Inventor: NAKAR, Elad, 2066717 Yokneam (IL); KEREN-TAL, Dor Zeev, 2066717 Yokneam (IL); ZIDAN, Laila Saleem, 2066717 Yokneam (IL); YANOVICH, Nir, 2066717 Yokneam (IL); BEN NATAN, Alon, 2066717 Yokneam (IL); KASSAR, Lotan Dvir, 2066717 Yokneam (IL)
(74) Representative: Carpmaels & Ransford LLP

(57) **Abstract**

A method includes receiving an anatomical map of wall tissue of at least a portion of a cardiac chamber, the map superimposed with a grid of ablation tags that are graphically encoded according to respective levels of ablation of the wall tissue. Upon placing a multi-electrode ablation catheter in a vicinity of the wall tissue, one or more of the ablation tags are graphically re-encoded according to (i) existing levels of ablation associated with the tags and (ii) electrode proximity to wall tissue locations associated with the tags. The anatomical map, having the re-encoded ablation tags, is displated to a user.

## Description

### FIELD OF THE DISCLOSURE

This disclosure relates generally to cardiac ablation, and specifically to a system and method for real-time planning and monitoring of cardiac ablation using an anatomical map.

### BACKGROUND OF THE DISCLOSURE

Providing indications of ablation points on an anatomical map of an inner wall of a cardiac chamber was previously proposed in the patent literature. For example, U.S. patent 9,757,182 describes a method including receiving locations of multiple ablation sites formed on the surface of a heart. Distances are measured among at least some of the ablation sites based on the locations. One or more gaps between the ablation sites, which meet an alerting criterion, are identified. The identified gaps are indicated to an operator.

As another example, U.S. patent 8,900,225 describes a method for performing a medical procedure that includes bringing a probe into contact with an organ in the body of a patient. A map of the organ is displayed, and the location of the probe relative to the map is tracked. A therapy is applied via the probe at multiple tissue sites in the organ with which the probe is brought into contact. The stability of the contact between the probe and the tissue sites is assessed while applying the therapy. The map is automatically marked, responsively to the assessed stability, to indicate the tissue sites at which the therapy was applied.

### BRIEF DESCRIPTION OF THE DRAWINGS

The present disclosure will be more fully understood from the following detailed description of the examples thereof, taken together with the drawings, in which:
Fig. 1 is a schematic, pictorial illustration of a catheter-based electroanatomical (EA) mapping and ablation system, in accordance with an example of the present disclosure;
Fig. 2 is an anatomical map of cardiac wall tissue superimposed with a grid of ablation tags graphically encoded according to the level of existing ablation, in accordance with an example of the present disclosure;
Fig. 3 is a schematic illustration of a graphical user interface (GUI) used for categorizing and graphically encoding the ablation tags of Fig. 2 according to the level of existing ablation, in accordance with an example of the present disclosure;
Figs. 4A and 4B are anatomical maps that show real-time graphical re-encoding of ablation tags, for identifying catheter electrodes suited to ablate wall tissue, in accordance with an example of the present disclosure; and
Fig. 5 is a flow chart that schematically illustrates a method for planning and monitoring ablation by graphically re-encoding ablation tags according to electrode proximity, in accordance with an example of the present disclosure.

### DETAILED DESCRIPTION OF EXAMPLES

### OVERVIEW

In a cardiac ablation procedure, such as pulmonary vein isolation (PVI) to treat atrial fibrillation, a physician ablates tissue in a specific anatomical region, (e.g., over an entire circumference of an ostium of a PV). The ablation, such as one using a pulsed-field ablation (PFA) technique, may require several iterations to cover the entire circumference of the ostium fully. Each iteration requires moving a multi-electrode ablation catheter to areas that are still insufficiently ablated, or were not ablated at all.

A physician attempting to complete the ablation may face several challenges, including (i) difficulty in closing ablation gaps, (ii) difficulty aligning a visualized 3D location with a previous ablation location, and (iii) preventing repetitive ablations on the same tissue location.

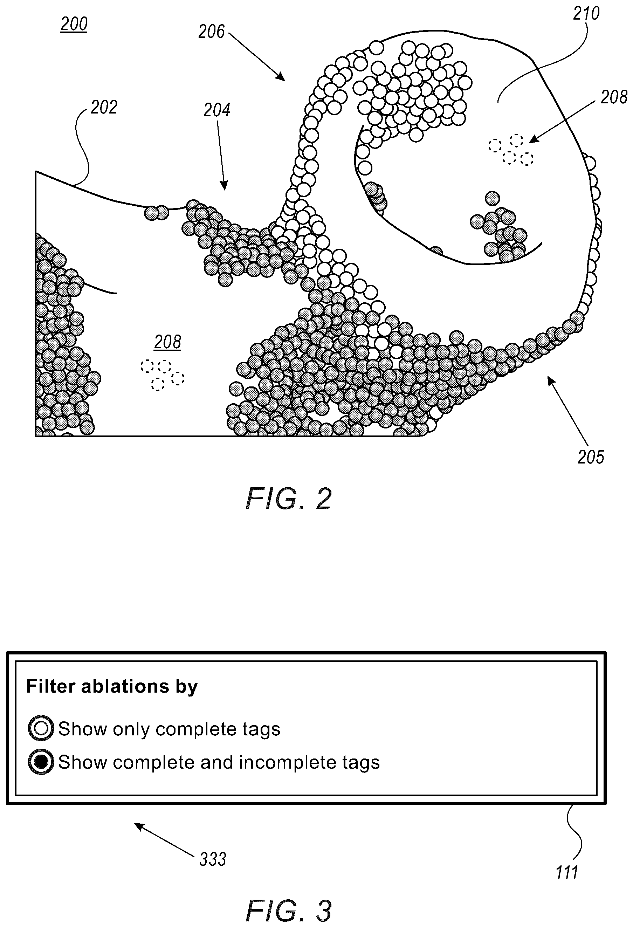

In the discussed application, the physician is typically aided with an anatomical 3D map that displays ablation tags (defined hereinafter as tags associated with grid ablation points as seen in Fig. 2), over the grid of the ablation points defined by the 3D mapping coordinate system. The grid of ablation points densely devides (e.g., sub-millimeter) the 3D space..

The disclosed ablation tags are located on the anatomical 3D map surface, including ones initially located sufficiently near the anatomical 3D map surface and accurately projected onto the surface. In an example, shown in Fig. 3, a graphical user interface (GUI) provided by the disclosed technique allows a user to choose to show graphically encoded ablation tags according to fully ablated and/or partially ablated (e.g., incompletely ablated) wall tissue locations.

The technique also displays new ablation tags on any ablation points respective tissue location not ablated before, when electrode proximity to wall tissue locations associated with the ablation points is below a given threshold.

Some examples of the present disclosure that are described herein provide a real-time visualization technique to aid a physician coping with the challenges, listed above, to make the correct decisions about where and how to perform further ablations.

In the disclosed real-time visualization technique, a processor receives an anatomical map of wall tissue of at least a portion of a cardiac chamber, the map superimposed with a grid of ablation tags that are graphically encoded according to respective levels of ablation of the wall tissue.

As the user (e.g., the physician) brings ablation electrodes of a multi-electrode or a single-electrode cathater in proximity to wall tissue, a processor re-encodes the ablation tags over the map according to (i) the existing level of ablation and (ii) electrode proximity to the tissue area. Based on the re-encoded ablation tags in the proximity of the electrode, the user may choose, for example, to ablate with a specific electrode, or to avoid ablation at that area.

If the specific electrode location overlaps an area that was either partially ablated or not ablated, the ablation tags in the area will, for example, show a new color (e.g., appear green). If the specific electrode overlaps an area that was previously fully ablated, the ablation tags will, for example, show a new color (e.g., appear red).

Different graphical encodings of the ablation grid points may be used. For example, as an alternative to color encoding of Fig. 2, the technique can use one shape, or another shape of icons, such as full and empty icons or circles and diamonds, for real-time marking of ablation tags over fully and partially ablated surface area locations, respectively.

The ablation tags may be superimposed on a 3D anatomical model which additionally displays another electrophysiological parameter, such as a Local Activation Time (LAT) 3D electroanatomical (EA) map.

Finally, the disclosed technique is applicable to a catheter located in space regardless of the 3D anatomy, such as in blood pool of a cardiac chamber. The volumetric grid of ablation points defined over the 3D space can still be tagged based on electrode proximity.

### SYSTEM DESCRIPTION

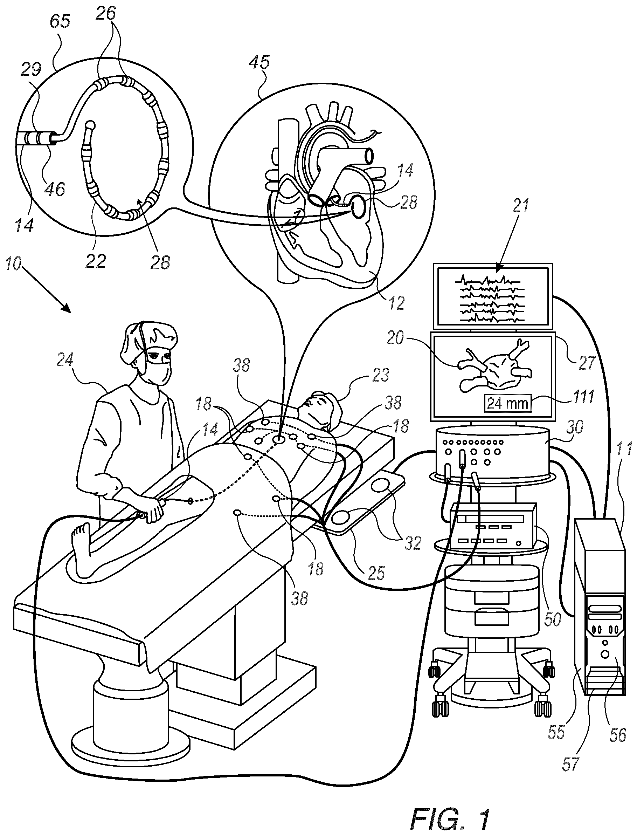

Fig. 1 is a schematic, pictorial illustration of a catheter-based electroanatomical (EA) mapping and ablation system 10, in accordance with an example of the present disclosure.

System 10 includes multiple catheters which are percutaneously inserted by physician 24 through the patient's vascular system into a chamber or vascular structure of a heart 12 (seen in inset 45). Typically, a delivery sheath catheter is inserted into a cardiac chamber, such as the left or right atrium near a desired location in heart 12. Thereafter, a plurality of catheters can be inserted into the delivery sheath catheter so as to arrive at the desired location. The plurality of catheters may include a catheter dedicated to pacing, a catheter for sensing intracardiac electrogram signals, a catheter dedicated to ablating and/or a catheter dedicated to both EA mapping and ablating. An example catheter 14, illustrated herein, is configured for sensing bipolar electrograms and Pulsed Field Ablation (PFA). Physician 24 brings a distal tip 28 (also called hereinafter distal end assembly 28) of catheter 14 into contact with the heart wall for ablating a target site in heart 12.

As seen in inset 65, catheter 14 is an exemplary catheter that includes a lasso distal end assembly 28, including one, and preferably multiple, electrodes 26 optionally distributed over a curved splne 22. Catheter 14 may additionally include a position sensor 29, embedded in or near distal tip 28 on a shaft 46 of catheter 14, to track the position and orientation of distal tip 28. Optionally, and preferably, position sensor 29 is a magnetic-based position sensor including three magnetic coils for sensing three-dimensional (3D) position and orientation.

The magnetic-based position sensor 29 may be operated together with a location pad 25 that includes a plurality of magnetic coils 32 configured to generate magnetic fields in a predefined working volume. The real-time position of distal tip 28 of catheter 14 may be tracked based on magnetic fields generated with location pad 25 and sensed by magnetic-based position sensor 29. Details of the magnetic-based position sensing technology are described in U.S. Patent Nos. 5,5391,199; 5,443,489; 5,558,091; 6,172,499; 6,239,724; 6,332,089; 6,484,118; 6,618,612; 6,690,963; 6,788,967; 6,892,091.

System 10 includes one or more electrode patches 38 positioned for skin contact on patient 23 to establish a location reference for location pad 25 as well as impedance-based tracking of electrodes 26. For impedance-based tracking, electrical current is directed toward electrodes 26 and sensed at electrode skin patches 38 so that the location of each electrode can be triangulated via electrode patches 38. Details of the impedance-based location tracking technology are described in US Patent Nos. 7,536,218; 7,756,576; 7,848,787; 7,869,865; and 8,456,182.

A recorder 11 displays cardiac signals 21 (e.g., electrograms acquired at respectively tracked cardiac tissue positions) acquired with body surface ECG electrodes 18 and intracardiac electrograms acquired with electrodes 26 of catheter 14. Recorder 11 may include pacing capability to pace the heart rhythm, and/or may be electrically connected to a standalone pacer.

System 10 may include an ablation energy generator 50 adapted to conduct ablative energy to one or more electrodes at a distal tip of a catheter configured for ablation. The energy produced by ablation energy generator 50 may include but is not limited to, radiofrequency (RF) energy or Pulse Field (PF) energy, including monopolar or bipolar and monophasic of biphasic high-voltage DC pulses, to be used to effect irreversible electroporation (IRE) or combinations thereof.

The patient interface unit (PIU) 30 is an interface configured to establish electrical communication between catheters, electrophysiological equipment, power supply, and a workstation 55 to control system 10 operation and receive EA signals from the catheter. Electrophysiological equipment of system 10 may include, for example, multiple catheters, location pad 25, body surface ECG electrodes 18, electrode patches 38, ablation energy generator 50, and recorder 11. Optionally, and preferably, PIU 30 additionally includes processing capability for implementing real-time computations of catheter locations and for performing ECG calculations.

Workstation 55 includes memory 57, a processor 56 unit with memory or storage with appropriate operating software loaded therein, and user interface capability. Workstation 55 may provide multiple functions, optionally including (i) modeling endocardial anatomy in three-dimensions (3D) and rendering the model or anatomical map 20 for display on a display device 27, (ii) displaying on display device 27 activation sequences (or other data) compiled from recorded cardiac signals 21 in representative visual indicia or imagery superimposed on the rendered anatomical map 20, (iii) displaying real-time location and orientation of multiple catheters within the heart chamber, and (iv) displaying sites of interest on display device 27 such as places where ablation energy has been applied. One commercial product embodying elements of system 10 is available as the CARTO^{™} 3 System, available from Biosense Webster, Inc., 31A Technology Drive, Irvine, CA 92618.

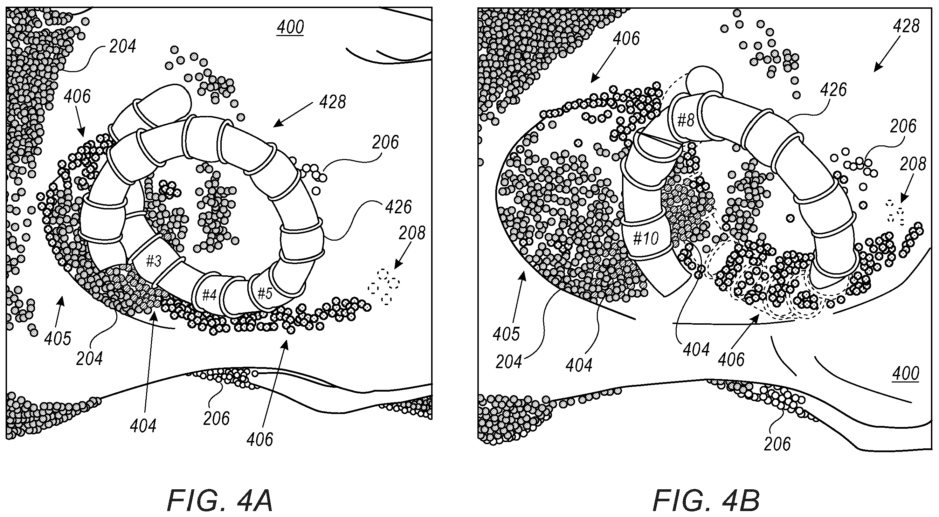

In the disclosed example, processor 56 runs an algorithm that presents physician 24 with regions of an anatomical map graphically re-encoded (e.g., recolored) in real time according to the existing level of ablation and ablation electrode proximity, as shown in Fig 4.

In some examples, processor 56 typically comprises a general-purpose computer programmed in software to carry out the functions described herein. The software may be downloaded to the computer in electronic form over a network, for example, or it may, alternatively or additionally, be provided and/or stored on non-transitory tangible media, such as magnetic, optical, or electronic memory.

This configuration of system 10 is shown by way of example, in order to illustrate certain problems that are addressed by examples of the present disclosure and to demonstrate the application of these examples in enhancing the performance of such a system. Examples of the present disclosure, however, are by no means limited to this specific sort of example system, and the principles described herein may similarly be applied to other sorts of medical systems. For example, other multi-electrode catheter types may be used, such as a basket catheter.

### GRAPHICAL ENCODING OF ABLATION GRID POINTS ACCORDING TO A LEVEL OF ABLATION

As noted above, the disclosed technique allows for graphically re-encoding grid ablation points on an anatomical map according to the existing level of ablation therein and the proximity of an ablation catheter electrode. Re-encoding is done on ablation tags that are already graphically encoded according to the existing level of ablation, including where no ablation was performed and whether or not the ablation was sufficient in accordance with parameters the physician defines, such as contact and position stability, for example.

Fig. 2 is an anatomical map 200 of cardiac wall tissue 202 superimposed with a grid of ablation tags 205 graphically encoded (204, 206) according to the existing level of ablation, in accordance with an example of the present disclosure. The dark shade 204 grid-based ablation tags indicate fully ablated areas. The pale shade 206 grid-based ablation tags indicate partially ablated areas (e.g., areas with incomplete ablation as identified using the physician's criteria). Dashed icons 208 stand for ablation points 208 over non-ablated areas that may have ablation tags displayed over there if catheter electrode is in sufficient proximity.

An ostium 210 surface of a PV shows partially or non-ablated regions that require further ablation. However, part of the ostium region also includes fully ablated areas. A physician attempting to complete the ablation of ostium 210 may face several challenges, including (i) difficulty in closing ablation gaps, (ii) difficulty aligning a visualized 3D location with a previous ablation location, and (iii) preventing repetitive ablation on the same tissue location.

Refering to Fig. 3, it is a schematic illustration of a graphical user interface (GUI) 111 used to categorize and graphically encode the ablation tags 205 of Fig. 2 according to the level of existing ablation, in accordance with an example of the present disclosure. Checkbox 333 on the GUI is used to select whether to show only fully ablated regions of wall tissue or both fully and partially ablated regions.

The disclosed real-time visualization technique, as seen in Fig. 2, re-encodes ablation tags 205 to guide a user of the 3D anatomical model about where to apply the upcoming ablation using selected electrodes.

### GRAPHICAL RE-ENCODING OF ABLATION GRID POINTS ACCORDING TO A LEVEL OF ABLATION AND ELECTRODE PROXIMITY

Figs. 4A and 4B are anatomical maps that show a real-time graphical re-encoding (404, 406) of ablation tags 405, for identifying catheter assembly 428 electrodes 426 suited to ablate wall tissue, in accordance with an example of the present disclosure.

In Figs. 4A and 4B, an ablation catheter, such as a loop catheter assembly 428 (e.g., VARIPULSE^{®} catheter provided by Biosense Webster) carrying electrodes 426 is shown brought in proximity with wall tissue to finalize an ablation. The processor graphically re-encodes (404, 406) only ablation tags 405 that are in sufficient proximity to any given electrode 426 (e.g., based on a tissue proximity index (TPI) algoritm described elsewhere).

Figs. 4A and 4B capture different real-time locations of the VARIPULSE^{®} catheter assembly 428, the two figures differing only by the identity of electrodes in relation to the wall tissue and in the respective real-time re-encoding layout of ablation tags 405.

Re-encoded tags 406 mark areas that require subsequent ablation either because no ablations were done in that area or insufficient ablations were done in that area. Re-encoded tags 404 mark areas that are already fully ablated, indicating to the user that electrode 426 is not required to apply further ablation in the vicinity. In the instance shown in Fig. 4A, electrode 3 should not be activated while electrode #4 and #5 should, while in the instance shown in Fig. 4B, electrode 10 should not be activated while electrode #8 should.

As further seen in Figs. 4A and 4B, grid ablation tags that are currently remote from ablation catheter 428 maintain their original graphical encodings (204, 206) since these are too remote of any electrode).

The processor displays one or more new ablation tags (405) on ablation points not ablated before, when electrode proximity to wall tissue locations associated with the ablation points is below a given threshold.

Finally, different graphical re-encodings (404, 406) of the ablation grid points may be used. In one example, as one can use color re-encoding of (404, 406) as (red, green). In another example, the technique can use one shape, or another shape of icons, such as (full, empty) icons or (circles, diamonds), for real-time marking of ablation tags over fully and partially or not ablated surface area locations, respectively.

### METHOD OF GRAPHICAL RE-ENCODING OF ABLATION GRID POINTS ACCORDING TO LEVEL OF ABLATION AND ELECTRODE PROXIMITY

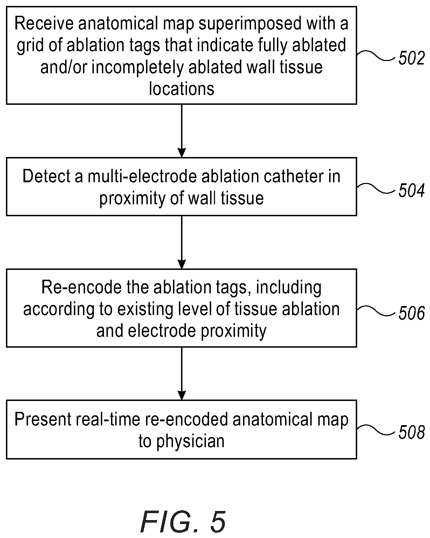

Fig. 5 is a flow chart that schematically illustrates a method for planning and monitoring grid-ablation by graphically re-encoding it with real-time ablation tags according to electrode proximity, in accordance with an example of the present disclosure. The algorithm, according to the presented example, carries out a process that begins with processor 56 receiving an anatomical map superimposed with a grid of ablation tags that indicate wall tissue locations already fully ablated and/or incompletely ablated, such as 3D map 200 at an ablation map receiving step 502.

Next, as the physician brings a multi-electrode ablation catheter into the proximity of wall tissue, the processor detects the level of electrode proximity to tissue at a pre-ablating step 504.

In response to detecting proximity, the processor re-encodes the ablation tags, according to the existing level (full, partial, none) of tissue ablation and according to each electrode's varying proximity to a tissue region, as seen in Figs. 4A and 4B, in re-encoding ablation tags step 506.

At map presenting step 508, the processor presents the user with the real-time re-encoded map to guide the physician about where to apply the upcoming ablation using which electrode.

### EXAMPLES

### Example 1

A method includes receiving an anatomical map (200) of wall tissue of at least a portion of a cardiac chamber, the map superimposed with a grid of ablation tags (205) that are graphically encoded (204, 206) according to respective levels of ablation of the wall tissue. Upon placing one of a multi-electrode (26, 28) or a single-electrode ablation catheter (14) in a vicinity of the wall tissue, one or more of the ablation tags (205) are graphically re-encoded (404, 406) according to (i) existing levels of ablation associated with the tags (205) and (ii) electrode (26) proximity to wall tissue locations associated with the tags (205). The anatomical map (400), having the re-encoded ablation tags (405), is displayed to a user.

### Example 2

The method according to example 1, and comprising displaying one or more new ablation tags (405) on ablation points (208) not ablated before, when electrode (26) proximity to wall tissue locations associated with the ablation points is below a given threshold, and displaying the anatomical map (400), having the one or more new ablation tags (405), to the user.

### Example 3

The method according to any of examples 1 and 2, wherein the levels of ablation are indicative of wall tissue locations that were fully ablated, partially ablated, or not ablated.

### Example 4

The method according to any of examples 1 through 3, wherein graphically re-encoding (404, 406) the ablation tags (405) comprises:
identifying an ablation tag (405) whose wall tissue location is predicted, based on the existing ablation level and the electrode proximity, to be damaged if further ablated; and
graphically re-encoding (404) the identified ablation tag (405) to indicate a warning.

### Example 5

The method according to any of examples 1 through 3, wherein graphically re-encoding (404, 406) the ablation tags (405) comprises:
identifying an ablation tag (405) whose wall tissue location is predicted, based on the existing ablation level and the electrode proximity, to remain safe if further ablated; and
graphically re-encoding (406) the identified ablation tag to indicate safe ablation.

### Example 6

The method according to any of examples 1 through 5, wherein the graphical re-encoding (404, 406) comprises using red color tags to mark fully ablation locations and green color tags to mark partially or not ablated locations.

### Example 7

The method according to any of examples 1 through 6, wherein the graphical re-encoding (404, 406) comprises using one shape of icon tags to mark fully ablation locations and another shape of icon tags to mark partially or not ablated locations.

### Example 8

The method according to claim any of examples 1 through 7, wherein graphically re-encoding (404, 406) the ablation tags is performed in real-time.

### Example 9

The method according to claim any of examples 1 through 8, wherein the anatomical map (200, 400) is an electroanatomical (EA) map.

### Example 10

A system includes an interface (30) and a processor (56). The interface (30) is configured to receive an anatomical map (200) of wall tissue of at least a portion of a cardiac chamber, the map superimposed with a grid of ablation tags (205) that are graphically encoded (204, 206) according to respective levels of ablation of the wall tissue. The processor (56) is configured to (i) upon placing one of a multi-electrode ablation catheter (26, 28) or a single-electrode ablation catheter (14) in a vicinity of the wall tissue, graphically re-encode (404, 406) one or more of the ablation tags according to (i) existing levels of ablation associated with the tags (205) and (ii) electrode proximity to wall tissue locations associated with the tags (205), and (ii) display the anatomical map (400), having the re-encoded ablation tags (404, 406), to a user.

Although the examples described herein mainly address cardiac diagnostic applications, the methods and systems described herein can also be used in other medical applications.

It will be appreciated that the examples described above are cited by way of example, and that the present disclosure is not limited to what has been particularly shown and described hereinabove. Rather, the scope of the present disclosure includes both combinations and subcombinations of the various features described hereinabove, as well as variations and modifications thereof which would occur to persons skilled in the art upon reading the foregoing description and which are not disclosed in the prior art.

## Claims

1. A system, comprising:
an interface (30) configured to receive an anatomical map (200) of wall tissue of at least a portion of a cardiac chamber, the map superimposed with a grid of ablation tags (205) that are graphically encoded (204, 206) according to respective levels of ablation of the wall tissue; and
a processor (56), which is configured to:
upon placing one of a multi-electrode (26, 28) or a single-electrode ablation catheter (14) in a vicinity of the wall tissue, graphically re-encode (404, 406) one or more of the ablation tags according to (i) existing levels of ablation associated with the tags (205) and (ii) electrode (26) proximity to wall tissue locations associated with the tags (205); and
display the anatomical map (400), having the re-encoded ablation tags (404, 406), to a user.

2. The system according to claim 1, wherein the processor (56) is further configured to display one or more new ablation tags (405) on ablation points (208) not ablated before when electrode (26) proximity to wall tissue locations associated with the ablation points is below a given threshold, and displaying the anatomical map (400), having the one or more new ablation tags (405), to the user.

3. The system according to claim 1 or claim 2, wherein the levels of ablation are indicative of wall tissue locations that were fully ablated, partially ablated, or not ablated.

4. The system according to any one of claims 1-3, wherein the processor (56) is configured to graphically re-encode the ablation tags by:
identifying an ablation tag (405) whose wall tissue location is predicted, based on the existing ablation level and the electrode proximity, to be damaged if further ablated; and
graphically re-encoding (404) the identified ablation tag (405) to indicate a warning.

5. The system according to any one of claims 1-4, wherein the processor (56) is configured to graphically re-encode the ablation tags by:
identifying an ablation tag (405) whose wall tissue location is predicted, based on the existing ablation level and the electrode proximity, to remain safe if further ablated; and
graphically re-encoding (406) the identified ablation tag to indicate safe ablation.

6. The system according to any one of claims 1-5, wherein the processor (56) is configured to graphically re-encode by using red color tags to mark fully ablation locations and green color tags to mark partially or not ablated locations.

7. The system according to any one of claims 1-6, wherein the processor (56) is configured to graphically re-encode by using one shape of icon tags to mark fully ablation locations and another shape of icon tags to mark partially or not ablated locations.

8. The system according to any one of claims 1-7, wherein the processor (56) is configured to graphically re-encode the ablation tags in real-time.

9. The system according to any one of claims 1-8, wherein the anatomical map (200, 400) is an electroanatomical (EA) map.
